# EUROPEAN PATENT APPLICATION

(11) **EP 1 731 153 A1**
(43) Date of publication of application: **13.12.2006**
(21) Application number: 05090166.9
(22) Date of filing: 07.06.2005
(51) Int. Cl.: A61K 31/502, A61K 9/16, A61K 9/20, A61K 9/28, A61P 35/00

(54) **Immediate-release and high-drug-load pharmaceutical formulations of non-micronised (4-chlorophenyl)¬4-(4-pyridylmethyl)phthalazin-1-yl| and salts thereof**

(71) Applicant: Schering Aktiengesellschaft, 13342 Berlin (DE)
(72) Inventor: Jürgens, Kai Christian, 13347 Berlin (DE); Backensfeld, Thomas, 13127 Berlin (DE); Funke, Adrian, 10587 Berlin (DE); Wagner, Torsten, 13127 Berlin (DE); Thode, Kai, 23795 Weede (DE)

(57) **Abstract**

The invention relates to immediate-release and high-drug-load solid pharmaceutical formulations comprising non- micronized (4-chlorophenyl)[4-(4-pyridylmethyl)-phthalazin-1-yl] as well as pharmaceutically acceptable salts thereof.

## Description

### FIELD OF THE INVENTION

The present invention relates to immediate-release and high-drug-load solid pharmaceutical formulations comprising (4-chlorophenyl)[4-(4-pyridylmethyl)-phthalazin-1-yl] as well as pharmaceutically acceptable salts thereof.

### BACKGROUND OF THE INVENTION

Two processes, namely the *de novo* formation of vessels from differentiating endothelial cells or angioblasts in the developing embryo (vasculogenesis) and the growth of new capillary vessels from existing blood vessels (angiogenesis), are involved in the development of the vascular systems of animal organs and tissues. Transient phases of new vessel formation (neovascularisation) also occur in the adult body, for example during the menstrual cycle, during pregnancy or during wound healing.

However, a number of diseases are known to be associated with deregulated angiogenesis, for example retinopathies, psoriasis, haemangioblastoma, haemangioma, and neoplastic diseases (solid tumours). Furthermore, the complex processes of vasculogenesis and angiogenesis have been found to involve a whole range of molecules, especially angiogenic growth factors and their endothelial receptors, as well as cell adhesion molecules.

Recent findings have shown that during embryonic development, during normal growth and in a wide number of pathological conditions and diseases, the angiogenic factor known as "Vascular Endothelial Growth Factor" (VEGF) forms part of the network regulating the growth and differentiation of the vascular system and its components (G. Breier et al., Trends in Cell Biology (1996) 6,454-456 and references cited therein).

VEGF, or more specifically VEGF-A, is a dimeric, disulfide-linked 46 kDa glycoprotein and is structurally related to "Platelet-Derived Growth Factor" (PDGF). It is produced by normal cell lines and tumour cell lines. VEGF is an endothelial cell-specific mitogen and shows angiogenic activity in *in vivo* test systems (e.g. rabbit cornea). VEGF is chemotactic for endothelial cells and monocytes, and induces plasminogen activators in endothelial cells, which are then involved in the proteolytic degradation of the extracellular matrix during formation of capillaries. A number of splice variants of VEGF-A are known which show comparable biological activity, but which differ in the type of cells that secrete them and in their heparin-binding capacity. In addition, there are other members of the VEGF family, such as "Placental Growth Factor" (PLGF), VEGF-B, VEGF-C and VEGF-D.

A large number of human tumours, especially gliomas and carcinomas, express high levels of the VEGF variants and their receptors. This has led to the hypothesis that the VEGF released by tumour cells could stimulate the growth of blood capillaries and the proliferation of tumour endothelium in a paracrine manner and thus, through the improved blood supply, accelerates tumour growth. Increased VEGF expression could explain the occurrence of cerebral oedema in patients with glioma. Direct evidence of the role of VEGF as a tumour angiogenesis factor *in vivo* has been obtained from studies in which VEGF expression or VEGF activity was inhibited. This was achieved with antibodies which inhibit VEGF activity, with dominant-negative VEGFR-2 mutants which inhibited signal transduction, as well as with use of antisense-VEGF RNA techniques. All approaches led to a reduction in the growth of glioma cell lines or other tumour cell lines *in vivo* as a result of inhibited tumour angiogenesis.

There are three VEGFR receptors with different affinities to the ligands. VEGF-A binds to VEGFR1 and VEGFR2; VEGF-B and Placental Growth Factor bind to VEGFR1; VEGF-A and processed forms of VEGF-C and VEGF-D bind to VEGFR-2; VEGF-C and VEGF-D bind to VEGFR-3.

VEGFR-3 is especially important for the growth of lymphatic vessels which play a role in tumour and metastases formation. Also in another diseases state, asthma, the lymphatic tissue is of major importance as it does remain in the alveoli after an acute inflammation and does not resolve like the blood vessels. This leads to the continuing susceptibility to stimulation by foreign agents.

All these receptors are transmembrane proteins. The signal of the VEGF variants is transmitted via the dimerisation of two receptor molecules inducing thereby an activation of the enzymatic activity at the intracellular C-terminal end. The enzymatic activity is a signal kinase, which transfers phosphates from ATP to itself (autophosphorylation) and to downstream signal molecules. This allows for interaction with an entire intracellular signal cascade eventually leading to endothelial cell proliferation and migration. The macroscopic result is the formation of new vessels.

WO 98/35958 describes generically a series of phthalazine derivatives with angiogenesis inhibiting activity and specifically (4-chlorophenyl)[4-(4-pyridylmethyl)-phthalazin-1-yl], including salts thereof, in particular the succinic acid salt thereof, as an interesting candidate for treatment of tumours. This compound is an inhibitor of all three VEGFR kinases. The inhibition is not dependent on a specific ligand, but blocks all the signals.

(4-chlorophenyl)[4-(4-pyridylmethyl)-phthalazin-1-yl] has the chemical structure set forth below:

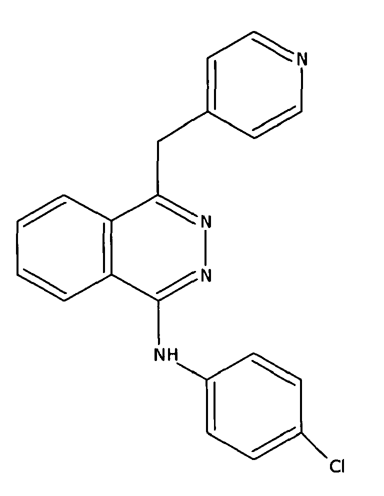

The solubility of (4-chlorophenyl)[4-(4-pyridylmethyl)-phthalazin-1-yl] is extremely dependent on pH. For example, the succinate salt of (4-chlorophenyl)[4-(4-pyridylmethyl)-phthalazin-1-yl] (hereinafter referred to as "pynasunate") has a reasonable solubility at very low pH values, whereas the solubility decreases significantly as the pH is increased:

| pH | Buffered | Solubility (mg/ml) | |
|---|---|---|---|
| | | 37°C | 20°C |
| 1.0 | no | 108 | |
| 1.1 | yes | | 83 |
| 2.0 | no | 146 | |
| 3.0 | yes | 7.9 | |
| 3.1 | yes | | 7.2 |
| 3.6 | no | 0.35 | |
| 3.7 | no | | 0.34 |
| 4.5 | yes | 0.02 | |
| 5.0 | yes | 3.7 x 10⁻³ | 2.9 x 10⁻³ |
| 7.0 | yes | 7.1 x 10⁻⁴ | 3.1 x 10⁻⁴ |

Since the drug substance is absorbed in the small intestine, where the pH is usually above 5, it is of utmost importance that essentially all of the drug substance is dissolved before entering the small intestine, i.e. essentially all of the drug substance should be dissolved in the gastric juice. Evidently, in order to obtain satisfactory absorption of the drug substance, the drug substance must be administered in an immediate-release formulation.

Traditionally, micronization has been used for the purpose of ensuring adequate release of a drug substance. However, in addition to the increased costs associated with the micronization procedure, a number of well-known manufacturing problems, such as agglomeration of the micronized particles, adherence of the micronized particles to production equipment, etc., may arise for the micronized drug substance. Furthermore, pynasunate is classified as being hazardous with an internal workspace limit of 0.1 mg/m³, i.e. handling of the drug substance must be under contained conditions and the use of e.g. high shear mixing granulation implies many product transfers and non-contained handlings of the drug substance.

Accordingly, there is a need for an immediate-release formulation based on non-micronized (4-chlorophenyl)[4-(4-pyridylmethyl)-phthalazin-1-yl], or pharmaceutically acceptable salts thereof, as well for as for processes for efficient preparation of such formulations.

In addition, clinical studies have revealed that about 1675 mg pynasunate have to be administered to cancer patients per day (in addition to other medicaments). The patients have to take about ten capsules a day which, in turn, leads to low patient compliance.

It is therefore evident that in addition to the need for a robust immediate-release formulation of the drug substance, there is also a need for a formulation having a high-drug-load.

The present inventors have surprisingly found that a formulation as described herein can be prepared with a robust prodcution process and which has a high load of non-micronized drug substance, an immediate-release profile and which contains a minimum of pharmaceutical excipients.

Thus, the object of the present invention is to provide a high-load solid pharmaceutical formulation of non-micronized (4-chlorophenyl)[4-(4-pyridylmethyl)-phthalazin-1-yl], or pharmaceutically acceptable salts thereof, which exhibits a reproducible immediate-release of the drug substance.

This object is met by the solid pharmaceutical formulations defined in the appended claims.

### SUMMARY OF THE INVENTION

In a first aspect the present invention relates to a solid pharmaceutical formulation comprising at least 50% by weight of the total formulation of non-micronized (4-chlorophenyl)[4-(4-pyridylmethyl)-phthalazin-1-yl], or a pharmaceutically acceptable salt thereof, wherein at least 70% of said (4-chlorophenyl)[4-(4-pyridylmethyl)-phthalazin-1-yl], or a pharmaceutical acceptable salt thereof, is dissolved from said solid pharmaceutical formulation within 30 minutes as determined by the USP 28 Paddle Method using 0.05 M KH₂PO₄/HCl buffer adjusted to pH 3.0 at 37°C as the dissolution media and 50 rpm as the stirring rate.

In a further aspect the present invention relates to a solid dosage form, in particular a solid unit dosage form, comprising the solid pharmaceutical formulation of the invention.

In a still further aspect the present invention relates to a composition of non-micronized particles of (4-chlorophenyl)[4-(4-pyridylmethyl)-phthalazin-1-yl], or a pharmaceutically acceptable salt thereof, having a d₉₀ value in the range of 50-300 µm and a d₅₀ value in the range of 15-100 µm, when determined as described herein.

In an even further aspect the present invention relates to the formulation of the invention, the dosage form of the invention, or the composition of the invention, for use as a medicament.

In another aspect the present invention relates to the use of the formulation of the invention, the dosage form of the invention, or the composition of the invention, for the manufacture of a medicament for the treatment of cancer.

In yet another aspect the present invention relates to a method of treating cancer, said method comprising administering a therapeutically effective amount of the formulation of the invention, the dosage form of the invention, or composition of the invention, to a patient in need thereof.

Further aspects of the present invention will be apparent from the below description and the appended claims.

### BRIEF DESCRIPTION OF THE DRAWINGS

Fig. 1 shows a particle size volume distribution of pynasunate prepared according to Example 1.
Fig. 2 shows a particle size volume distribution of pynasunate prepared according to Example 2.
Fig. 3 shows a particle size volume distribution of pynasunate prepared according to Example 3.
Fig. 4 shows a particle size volume distribution of pynasunate prepared according to Example 4.
Fig. 5 shows the relationship between compression force and hardness of the tablets prepared in Example 6.

### DETAILED DESCRIPTION OF THE INVENTION

As explained above, the present invention provides an immediate-release and high-load solid pharmaceutical formulation comprising the drug substance (4-chlorophenyl)[4-(4-pyridylmethyl)-phthalazin-1-yl], or a pharmaceutically acceptable salt thereof, in non-micronized form.

### Solid pharmaceutical formulation

When used herein, the term "non-micronized" is intended to mean that the particle size distribution is so that at least 90% of the particles have a particle diameter of more than 50 µm (calculated from the volume distribution curve under the presumption of spherical particles using laser diffraction methods), i.e. a d₉₀ value of at least 50 µm. It is well-known to the skilled person that parameters used to describe a given particle size distribution may vary considerably dependent on the specific equipment and settings used. Therefore, it is important to note that whenever the terms "particle size distribution", "particle diameter", "d₅₀", "d₉₀", "d₉₅", "d₉₉", etc. are used herein it should be understood that the specific values or ranges used in connection therewith are always meant to be determined from the volume distribution curve under the presumption of spherical particles using laser diffraction and the conditions set forth in the section entitled "Determination of particle size distribution" herein.

In one embodiment of the invention the d₉₀ value of (4-chlorophenyl)[4-(4-pyridylmethyl)-phthalazin-1-yl], or a pharmaceutically acceptable salt thereof, is at least 60 µm, such as at least 70 µm, e.g. at least 80 µm, or at least 90 µm. In a highly preferred embodiment of the invention the d₉₀ value is at least 100 µm, such as at least 110 µm, e.g. at least 120 µm, more preferably at least 130 µm, such as at least 140 µm. In addition, the d₅₀ value of (4-chlorophenyl)[4-(4-pyridylmethyl)-phthalazin-1-yl], or a pharmaceutically acceptable salt thereof, should preferably be at least 15 µm, in particular at least 20 µm, such as at least 25 µm, e.g. at least 30 µm, most preferably at least 35 µm.

As will be understood from the examples provided herein, the present invention is, at least in part, based on the surprising discovery that stable, immediate-release formulations may be prepared even without micronizing the drug substance. On the other hand, it is also evident from the examples provided herein that the particle size of the drug substance should not be too large as the dissolution properties are then impeded. Accordingly, the d₉₀ value of (4-chlorophenyl)[4-(4-pyridylmethyl)-phthalazin-1-yl], or a pharmaceutically acceptable salt thereof, will typically be in the range of 50-300 µm. In a preferred embodiment of the invention, the d₉₀ value is in the range of 60-250 µm, such as in the range of 70-200 µm, e.g. in the range of 80-200 µm, more preferably in the range of 90-200 µm, such as in the range of 100-200 µm, e.g. in the range of 110-190 µm, most preferably in the range of 120-180 µm, such as in the range of 130-170 µm, e.g. in the range of 140-160 µm. Analogously, the d₅₀ value of (4-chlorophenyl)[4-(4-pyridylmethyl)-phthalazin-1-yl], or a pharmaceutically acceptable salt thereof, will typically be in the range of 15-100 µm, in particular in the range of 20-90 µm, such as in the range of 25-80 µm, e.g. in the range of 30-70 µm.

As will be understood from the present disclosure, including the examples provided herein, it is of utmost importance that the drug substance is released in a fast and reliable manner under acidic conditions. Thus, in the present context, the terms "fast-release" or "immediate-release" mean that at least 70% of the drug substance (i.e. (4-chlorophenyl) [4-(4-pyridylmethyl)-phthalazin-1-yl] or a pharmaceutically acceptable salt thereof) is dissolved from the solid pharmaceutical formulation within 30 minutes as determined by the USP 28 Paddle Method using 0.05 M KH₂PO₄/HCl buffer adjusted to pH 3.0 at 37°C as the dissolution media and 50 rpm as the stirring rate. In a preferred embodiment of the invention at least 75%, more preferably at least 80%, of the drug substance is dissolved from the solid pharmaceutical formulation within 30 minutes as determined by the USP 28 Paddle Method described herein.

As will also be understood from the present disclosure it is of utmost importance that the solid pharmaceutical formulation contains a "high-load" of drug substance due to the relative large amount of drug substance needed for administration to patients. Thus, in the present context, the term "high-load" or "high-drug-load" means that the solid pharmaceutical formulation contains at least 50% by weight, calculated on the basis of the total weight of the formulation, of the drug substance (i.e. (4-chlorophenyl) [4-(4-pyridylmethyl)-phthalazin-1-yl] or a pharmaceutically acceptable salt thereof). In an interesting embodiment of the invention the solid pharmaceutical formulation comprises at least 55% by weight, such as at least 60% by weight, e.g. at least 65% by weight, preferably at least 70% by weight, such as at least 75% by weight, e.g. at least 80% by weight, at least 85% by weight, at least 90% by weight, or at least 90% by weight of the drug substance, calculated on the basis of the total weight of the formulation. Stated differently, the solid pharmaceutical formulation typically comprises 50-95% by weight of the drug substance (i.e. (4-chlorophenyl) [4-(4-pyridylmethyl)-phthalazin-1-yl] or a pharmaceutically acceptable salt thereof), calculated on the basis of the total weight of the formulation. In an interesting embodiment of the invention the solid pharmaceutical formulation comprises 50-70% by weight or 70-90% by weight, such as 55-65% by weight or 85-95% by weight of the drug substance, calculated on the basis of the total weight of the formulation.

The drug substance itself may be the free base, i.e. (4-chlorophenyl)[4-(4-pyridylmethyl)-phthalazin-1-yl] or a pharmaceutically acceptable salt thereof, in particular an acid addition salt. Such salts may be formed from suitable inorganic or organic acids. Examples of suitable inorganic acids include the halogen acids, such as hydrochlorid acid; sulfuric acid; and phosphoric acid. Examples of suitable organic acids include carboxylic, phosphonic, sulfonic or sulfamic acids, such as acetic acid, propionic acid, octanoic acid, decanoic acid, dodecanoic acid, glycolic acid, lactic acid, 2-hydroxybutyric acid, gluconic acid, glucose monocarboxylic acid, fumaric acid, succinic acid, adipic acid, pimelic acid, suberic acid, azelaic acid, malic acid, tartaric acid, citric acid, glucaric acid, galactaric acid; amino acids, such as glutamic acid, aspartic acid, N-methylglycine, acetylaminoacetic acid, N-acetyl-asparagine and N-acetylcysteine; pyruvic acid, acetoacetic acid, phosphoserine, 2- or 3-glycerophosphoric acid, glucose-6-phosphoric acid, glucose-1-phosphoric acid, fructose-1,6-bis-phosphoric acid, maleic acid, hydroxymaleic acid, methylmaleic acid, cyclohexanecarboxylic acid, adamantanecarboxylic acid, benzoic acid, salicylic acid, 1- or 3-hydroxynaphthyl-2-carboxylic acid, 3,4,5-trimethoxybenzoic acid, 2-phenoxybenzoic acid, 2-acetoxybenzoic acid, 4-aminosalicylic acid, phthalic acid, phenylacetic acid, mandelic acid, cinnamic acid, glucuronic acid, galacturonic acid, methane- or ethane-sulfonic acid, 2-hydroxyethanesulfonic acid, ethane-1,2-disulfonic acid, 2-, 3-, or 4-methylbenzenesulfonic acid, methylsulfuric acid, ethylsulfuric acid, dodecylsulfuric acid, N-cyclohexylsulfamic acid, N-methyl-, N-ethyl-, or N-propyl-sulfamic acid, or other organic acids, such as ascorbic acid.

In the most preferred embodiment of the invention the drug substance is the succinate salt of (4-chlorophenyl)[4-(4-pyridylmethyl)-phthalazin-1-yl].

Herein, the term "pynasunate" refers to the succinate salt of (4-chlorophenyl)[4-(4-pyridylmethyl)-phthalazin-1-yl], i.e. (4-chlorophenyl)[4-(4-pyridylmethyl)-phthalazin-1-yl]ammonium hydrogen succinate. Pynasunate is described in WO 98/35958.

The solid pharmaceutical formulation of the invention may, in addition to non-micronized (4-chlorophenyl)[4-(4-pyridylmethyl)-phthalazin-1-yl], or a pharmaceutically acceptable salt thereof, contain at least one pharmaceutically acceptable excipient. Such excipients may, e.g., be selected from the group consisting of fillers, binders, surfactants, disintegrants, glidants and lubricants.

Specific examples of such pharmaceutically acceptable excipients include be:
- Inert diluents or fillers,
   such as sucrose, sorbitol, sugars, mannitol, microcrystalline cellulose, starches, polysaccharides, carragenan, lamda-carragenan, kappa-carragenan, iota-carragenan, sodium chloride, sodium phosphate, calcium carbonate, calcium phosphate, calcium sulfate or lactose, e.g. lactose monohydrate. The inert diluent or filler is typically present in an amount from 1-50% by weight of the total formulation. Preferably, the inert diluent or filler is present in an amount from 1-40% by weight of the total formulation. In one particular interesting embodiment of the invention, the inert diluent or filler is present in an amount from 1-20% by weight of the total formulation, preferably in an amount from 1-10% by weight of the total formulation, more preferably in an amount from 1-7.5% by weight of the total formulation, most preferably in an amount from 1-5% by weight of the total formulation. In another particular interesting embodiment of the invention, the inert diluent or filler is present in an amount from 20-40% by weight of the total formulation, preferably in an amount from 25-35% by weight of the total formulation, more preferably in an amount from 30-35% by weight of the total formulation. In a preferred embodiment of the invention, the inert filler or diluent is lactose, in particular lactose monohydrate.
- Binders,
   such as sucrose, glucose, sorbitol, acacia, alginic acid, sodium alginate, gelatine, starch, pregelatinised starch, microcrystalline cellulose, polysaccharides, carragenan, lamda-carragenan, kappa-carragenan, iota-carragenan, magnesium aluminium silicate, carboxymethylcellulose sodium (CMC sodium), methylcellulose, ethylcellulose, hydroxypropylmethylcellulose (HPMC), polyvinylacetate, polyethylene glycol or polyvinylpyrrolidone, e.g. PVP K12, PVP K15, PVP K17, PVP K25, PVP K30, PVP K60, PVP K90, PVP K120 or combinations thereof. The binder is typically present in an amount from 0.5-15% by weight of the total formulation. Preferably, the binder is present in an amount from 1-10% by weight of the total formulation, more preferably in an amount from 1-5% by weight of the total formulation. In a preferred embodiment of the invention, the binder is HPMC, in particular HPMC having viscosity grade 3.
- Lubricants, including glidants and antiadhesives,
   such as magnesium stearate, zinc stearate, stearic acid, silicas, hydrogenated vegetable oils or talc. The lubricant is typically present in an amount from 0.1-10% by weight of the total formulation. Preferably, the lubricant is present in an amount from 0.2-5% by weight of the total formulation, such as from 0.5-5% by weight of the total formulation, e.g. from 1-5% by weight of the total formulation, more preferably in an amount from 1-3% by weight of the total formulation. In a preferred embodiment of the invention, the lubricant is magnesium stearate.
- Disintegrants,
   such as croscarmellose sodium, cross-linked povidone, sodium starch glycolate, maize starch or potato starch. The disintegrant is typically present in an amount from 1-20% by weight of the total formulation. Preferably, the disintegrant is present in an amount from 1-15% by weight of the total formulation, such as from 1-10% by weight of the total formulation, e.g. from 1-7.5% by weight of the total formulation, more preferably from 1-5% by weight of the total formulation. In a preferred embodiment of the invention, the disintegrant is croscarmellose sodium. Croscarmellose sodium is commercially available under the trademark Ac-Di-Sol^{®}.
- Surfactants and wetting agents,
   such as naturally occurring phosphatides, e.g. lechitin or soybean lechitin; condensation products of ethylene oxide with e.g. a fatty acid, a long chain fatty alcohol, or a partial ester derived from fatty acids and a hexitol or a hexitol anhydride, for example polyoxyethylene stearate, polyoxyethylene sorbitol monooleate, polyoxyethylene sorbitan monooleate, etc.; or salts of long-chain aliphatic phosphates, such as sodium lauryl sulphate.

Examples of other pharmaceutically acceptable excipients which may be incorporated in the solid pharmaceutical formulation of the invention include colorants, flavouring agents, plasticizers, humectants, buffering agents, etc.

The solid pharmaceutical formulations of the invention may also contain further drug substances, such as anti-cancer agents.

In those cases where the pharmaceutical formulation is in the form of a solid dosage form, in particular a solid unit dosage form (e.g. a tablet, sachet or capsule, in particular a tablet), the unit dosage form is adapted for oral administration and may be provided with a coating, such as a film coating, a sugar coating, or the like. Thus, a suitable coating for the solid unit dosage form according to the invention may, for example, be a sugar coating or a film coating based on one or more of the ingredients: Hydroxypropylmethylcellulose (HPMC), methylcellulose, ethylcellulose, hydroxyethylmethylcellulose, hydroxypropylcellulose, carboxymethylcellulose sodium, acrylate polymers (e.g.
Eudragit^{®}), polyethylene glycols or polyvinylpyrrolidone. Preferably, the coating is a film coating based on HPMC.

The coated or uncoated tablet typically has a weight in the range from 500-700 mg, such as in the range of 525-675 mg, e.g. in the range of 525-650 mg. In one particular interesting embodiment of the invention, the coated or uncoated tablet has a weight in the range of 525-575 mg, such as about 550 mg if uncoated or about 562 mg if coated. In another particular interesting embodiment of the invention, the coated or uncoated tablet has a weight in the range of 610-650 mg, such as about 627 mg if uncoated or about 640 mg if coated.

The amount of active drug substance, in particular (4-chlorophenyl)[4-(4-pyridylmethyl)-phthalazin-1-yl]ammonium hydrogen succinate, present in the tablet will typically be in the range from 300-600 mg. In one particular interesting embodiment of the invention, the tablet comprises 300-350 mg, such as 310-350 mg, e.g. 320-350 mg, preferably 330-340 mg, more preferably about 335 mg drug substance. In another particular interesting embodiment of the invention, the tablet comprises 500-600 mg, such as 510-600 mg, e.g. 520-600 mg, preferably 530-590 mg, such as 540-580 mg, e.g. 550-570, more preferably 555-565 mg, such as about 558.3 mg drug substance.

In an interesting embodiment of the invention, the uncoated tablets contain the following ingredients:

| Ingredient | % |
|---|---|
| Drug substance (e.g. pynasunate) | 50-95 |
| Filler (e.g. lactose monohydrate, mannitol) | 1-50 |
| Binder (e.g. HPMC, microcrystalline cellulose) | 1-10 |
| Disintegrant (e.g. croscamellose sodium) | 1-20 |
| Lubricant (e.g. magnesium stearate) | 0.1-10 |

The respective uncoated tablets may be coated.

In a preferred embodiment of the invention, the uncoated tablets contain the following ingredients:

| Ingredient | % | % |
|---|---|---|
| Drug substance (e.g. pynasunate) | 50-70 | 70-90 |
| Filler (e.g. lactose monohydrate, mannitol) | 20-40 | 1-20 |
| Binder (e.g. HPMC, microcrystalline cellulose) | 1-5 | 1-5 |
| Disintegrant (e.g. croscamellose sodium) | 1-5 | 1-5 |
| Lubricant (e.g. magnesium stearate) | 1-5 | 1-5 |

The respective uncoated tablets may be coated.

In an even more preferred embodiment of the invention, the uncoated tablets contain the following ingredients:

| Ingredient | % | % |
|---|---|---|
| Drug substance (e.g. pynasunate) | 55-65 | 85-95 |
| Filler (e.g. lactose monohydrate, mannitol) | 25-35 | 1-10 |
| Binder (e.g. HPMC, microcrystalline cellulose) | 1-5 | 1-5 |
| Disintegrant (e.g. croscamellose sodium) | 1-5 | 1-5 |
| Lubricant (e.g. magnesium stearate) | 1-3 | 1-3 |

The respective uncoated tablets may be coated.

In the most preferred embodiment of the invention, the uncoated tablets contain the following ingredients:

| Ingredient | %* | %* | %** | %** |
|---|---|---|---|---|
| Drug substance (e.g. pynasunate) | 60.9 | 89.0 | 59.6 | 87.2 |
| Filler (e.g. lactose monohydrate, mannitol) | 32.0 | 2.9 | 31.3 | 2.8 |
| Binder (e.g. HPMC, microcrystalline cellulose) | 2.5 | 2.5 | 2.6 | 2.5 |
| Disintegrant (e.g. croscamellose sodium) | 2.7 | 3.7 | 2.7 | 3.6 |
| Lubricant (e.g. magnesium stearate) | 1.8 | 1.8 | 1.8 | 1.8 |

The respective uncoated tablets may be coated.

Stated differently, the uncoated tablets may, in a typical embodiment of the invention, contain the following ingredients:

| Ingredient | Amount (mg) |
|---|---|
| Drug substance (e.g. pynasunate) | 300-600 |
| Filler (e.g. lactose monohydrate, mannitol) | 5-250 |
| Binder (e.g. HPMC, microcrystalline cellulose) | 5-50 |
| Disintegrant (e.g. croscamellose sodium) | 5-100 |
| Lubricant (e.g. magnesium stearate) | 0.5-25 |

The respective uncoated tablets may be coated.

In a preferred embodiment of the invention, the uncoated tablets contain the following ingredients:

| Ingredient | Amount (mg) | Amount (mg) |
|---|---|---|
| Drug substance (e.g. pynasunate) | 300-350 | 500-600 |
| Filler (e.g. lactose monohydrate, mannitol) | 110-220 | 1-50 |
| Binder (e.g. HPMC, microcrystalline cellulose) | 5-30 | 5-30 |
| Disintegrant (e.g. croscamellose sodium) | 5-35 | 5-35 |
| Lubricant (e.g. magnesium stearate) | 1-20 | 1-20 |

The respective uncoated tablets may be coated.

In a more preferred embodiment of the invention, the uncoated tablets contain the following ingredients:

| Ingredient | Amount (mg) | Amount (mg) |
|---|---|---|
| Drug substance (e.g. pynasunate) | 320-350 | 540-580 |
| Filler (e.g. lactose monohydrate, mannitol) | 150-200 | 10-30 |
| Binder (e.g. HPMC, microcrystalline cellulose) | 5-25 | 5-25 |
| Disintegrant (e.g. croscamellose sodium) | 5-25 | 10-30 |
| Lubricant (e.g. magnesium stearate) | 5-15 | 5-15 |

The respective uncoated tablets may be coated.

In an even more preferred embodiment of the invention, the uncoated tablets contain the following ingredients:

| Ingredient | Amount (mg) | Amount (mg) |
|---|---|---|
| Drug substance (e.g. pynasunate) | 330-340 | 555-565 |
| Filler (e.g. lactose monohydrate, mannitol) | 170-180 | 15-20 |
| Binder (e.g. HPMC, microcrystalline cellulose) | 10-20 | 10-20 |
| Disintegrant (e.g. croscamellose sodium) | 10-20 | 20-30 |
| Lubricant (e.g. magnesium stearate) | 5-15 | 5-15 |

The respective uncoated tablets may be coated.

In a still more preferred embodiment of the invention, the uncoated tablets contain the following ingredients:

| Ingredient | Amount (mg) | Amount (mg) |
|---|---|---|
| Drug substance (e.g. pynasunate) | 332-337 | 557-560 |
| Filler (e.g. lactose monohydrate, mannitol) | 174-178 | 17-19 |
| Binder (e.g. HPMC, microcrystalline cellulose) | 13-15 | 15-17 |
| Disintegrant (e.g. croscamellose sodium) | 15-16 | 22-25 |
| Lubricant (e.g. magnesium stearate) | 9-11 | 10-13 |

The respective uncoated tablets may be coated.

In the most preferred embodiment of the invention, the uncoated tablets contain the following ingredients:

| Ingredient | Amount (mg) | Amount (mg) |
|---|---|---|
| Drug substance (e.g. pynasunate) | 335 | 558.3 |
| Filler (e.g. lactose monohydrate, mannitol) | 176 | 18 |
| Binder (e.g. HPMC, microcrystalline cellulose) | 14 | 16 |
| Disintegrant (e.g. croscamellose sodium) | 15 | 23.2 |
| Lubricant (e.g. magnesium stearate) | 10 | 11.5 |

The respective uncoated tablets may be coated.

In another embodiment of the invention, granules contain ingredients according to anyone of the preceding compositions wherein the lubricant is left out.

In an other interesting embodiment of the invention the granule is shaped to pellets, whereas the respective granule contains the following ingredients:

| Ingredient | Amount (%) | Amount (mg) |
|---|---|---|
| Drug substance (e.g. pynasunate) | 87.3 | 1675 |
| Filler (e.g. lactose monohydrate, mannitol, carragenan) | 2.7 | 52 |
| Binder (e.g. HPMC, microcrystalline cellulose, carragenan) | 5.0 | 96 |
| Disintegrant (e.g. croscamellose sodium) | 5.0 | 96 |
| Lubricant (e.g. magnesium stearate) | 0 | |

The respective pellets may be coated.

The solid pharmaceutical formulation and the solid unit dosage form of the invention is typically prepared by means of a granulation process, i.e. the non-micronized drug substance, together with appropriate excipients, is subjected to a granulation process, preferably a wet granulation process, such as a fluid bed granulation process. After the granulation process, the granules are processed further into the final solid unit dosage form. In one embodiment of the invention the granules may be filled in capsules, such as hard gelatine capsules. However, in a preferred embodiment of the invention the granules are processed into tablets by compression and subsequently film-coated.

Accordingly, the present invention is also directed to a process for the preparation of granules comprising at least 50% by weight of the total granule of non-micronized (4-chlorophenyl)[4-(4-pyridylmethyl)-phthalazin-1-yl], or a pharmaceutically acceptable salt thereof, said process comprising the steps of
i) preparing a liquid medium comprising the binder
ii) subjecting a powder mixture of excipients such as filler and diluents and at least 50% by weight of the components of non-micronized (4-chlorophenyl)[4-(4-pyridylmethyl)-phthalazin-1-yl], or a pharmaceutically acceptable salt thereof to a granulator
iii) subjecting said liquid medium to a granulation process; and
iv) optionally extruding and shaping the granules
v) optionally drying the granules
vi) optionally adding further excipients such as disintegrants and lubricants to the granules
vii) optionally collecting the granules.

In a related aspect, the present invention is also directed to a process for the preparation of a solid unit dosage form according to the invention, said process comprising the steps of
i) preparing granules comprising at least 50% by weight of the total granule of non-micronized (4-chlorophenyl)[4-(4-pyridylmethyl)-phthalazin-1-yl], or a pharmaceutically acceptable salt thereof, as described above;
ii) formulating said granules into solid unit dosage forms.

The liquid medium typically comprises a binder. In an interesting embodiment of the invention, the liquid does not contain a disintegrant and/or a lubricant. Preferably, the disintegrant and/or the lubricant is added during the granulation process, in particular at the end of the granulation process. The liquid medium is preferably water.

The powder blend typically comprises at least 55% by weight of the components of the medium, excluding liquid, of non-micronized (4-chlorophenyl)[4-(4-pyridylmethyl)-phthalazin-1-yl], or a pharmaceutically acceptable salt thereof. In a preferred embodiment of the invention the powder blend comprises at least 60% by weight, such as at least 65% by weight, e.g. at least 70% by weight, preferably at least 75% by weight, such as at least 80% by weight, e.g. at least 85% by weight, at least 90% by weight or at least 95% by weight of the components of non-micronized (4-chlorophenyl)[4-(4-pyridylmethyl)-phthalazin-1-yl], or a pharmaceutically acceptable salt thereof.

As can be seen from the Examples provided herein, a number of batches, were manufactured using pynasunate having a varying particle size distribution.

It was found that very fine (micronized) particles of the pynasunate raw material led to considerably manufacturing problems. Compression of the granules formed from the micronized pynasunate particles was not possible as the thereby formed tablets exhibited sticking to the punches and decreased physical stability. In addition, it was observed that the granules prepared from micronized pynasunate particles were less coarse, i.e. the lower the particle size of the micronized pynasunate particles, the lower the amount (in percentage) of granules having a particle size above 300 µm.

Non-micronized particles of pynasunate exhibited good to excellent compressibility properties. Only few or no problems with respect to sticking to the punches were encountered. Furthermore, tablets prepared from non-micronized pynasunate particles showed satisfactory physical stability and satisfactory immediate-release of pynasunate. However, the particle size of the granules should not be too large as the release properties are then impeded.

In an interesting embodiment of the invention the granules obtainable by the process described herein are so that least 25% of the granules are coarser than 250 µm as determined by sieve analysis in accordance with Ph. Eur. Method 2.9.12 using a sieve with a mean mesh width of 250 µm. Preferably, at least 30% of the granules, such as at least 35% of the granules, e.g. at least 40% of the granules are coarser than 250 µm. More preferably, 25-70% of the granules are coarser than 250 µm as determined by sieve analysis in accordance with Ph. Eur. Method 2.9.12 using a sieve with a mean mesh width of 250 µm. In particular 30-70% of the granules, such as 35-70% of the granules, e.g. 40-70% of the granules, most preferably 40-65% of the granules, such as 40-60% of the granules are coarser than 250 µm.

### Non-micronized pynasunate

As described above, the present inventors have surprisingly found that non-micronized pynasunate exhibits excellent manufacturing properties allowing high-load solid pharmaceutical formulations to be prepared. Accordingly, the present invention is also directed to a composition of non-micronized (4-chlorphenyl)[4-(4-pyridylmethyl)-phtalazin-1-yl]ammonium hydrogen succinate particles having a d₉₀ value in the range of 50-300 µm and a d₅₀ value in the range of 15-100 µm, when determined as described herein. In a preferred embodiment, the d₉₀ value is in the range of 100-200 µm and the d₅₀ value is in the range of 30-70 µm, when determined as described herein. In a highly preferred embodiment of the invention, the composition of non-micronized (4-chlorphenyl)[4-(4-pyridylmethyl)-phtalazin-1-yl]ammonium hydrogen succinate particles has the following d₅₀, d₉₀, d₉₅ and d₉₉ values:

| Composition | d₅₀ | d₉₀ | d₉₅ | d₉₉ |
|---|---|---|---|---|
| A1 | 30-50 | 100-140 | 150-180 | 260-300 |
| A2 | 35-45 | 110-130 | 155-175 | 270-290 |
| A3 | 40-44 | 117-121 | 162-166 | 280-284 |
| | | | | |
| B1 | 25-50 | 120-160 | 160-200 | 210-250 |
| B2 | 30-40 | 130-150 | 165-185 | 220-240 |
| B3 | 35-40 | 139-143 | 172-176 | 227-231 |
| | | | | |
| C1 | 45-75 | 150-190 | 180-220 | 225-265 |
| C2 | 55-70 | 160-180 | 190-210 | 235-255 |
| C3 | 60-65 | 165-175 | 200-205 | 245-250 |

| | | | | |
|---|---|---|---|---|
| All values in µm | | | | |

In a particular interesting embodiment of the invention the composition of non-micronized (4-chlorphenyl)[4-(4-pyridylmethyl)-phtalazin-1-yl]ammonium hydrogen succinate particles has the d₅₀, d₉₀, d₉₅ and d₉₉ values shown in Example 1, Example 2 or Example 3 herein.

### Medical use

The present invention provides means and methods for treating certain cancers or tumours or tumour angiogenesis, in any suitable animal, preferably in a mammal, and in particular in a human being.

The term "solid malignant tumor" is intended to indicate an abnormal malignant mass of tissue that is not inflammatory, which arises without obvious cause from cells of preexistent tissue, which possesses no physiologic function and which has the ability to invade normal cells and spread throughout the body. Examples of typical solid malignant tumours include breast carcinoma, non-small cell lung cancer, colon carcinoma, renal cell carcinoma and malignant melanoma.

The term "carcinoma" is intended to indicate a malignant tumour of epithelial origin. Epithelial tissue covers or lines the body surfaces inside and outside the body. Examples of epithelial tissue are the skin and the mucosa and serosa that line the body cavities and internal organs, such as intestines, urinary bladder, uterus, etc. Epithelial tissue may also extend into deeper tissue layers to from glands, such as mucus-secreting glands.

The term "sarcoma" is intended to indicate a malignant tumour growing from connective tissue, such as cartilage, fat, muscles, tendons and bones.

The term "leukaemia" is intended to indicate a blood cancer, i.e. a cancer that originates from the bone marrow and which keeps the marrow from producing normal red and white blood cells and platelets.

The term "lymphoma" refers to a cancer that originates in the nodes or glands of the lymphatic system.

The term "glioma", when used herein, is intended to cover a malignant tumor originating from glial cells.

The term "inhibition" or "inhibit" as used in connection with treatment of solid tumours is intended to cover the delayed appearance of primary or secondary tumours, slowed development of primary or secondary tumours, decreased occurrence of primary or secondary tumours, slowed or decreased severity of secondary effects of disease, arrested tumour growth and regression of tumours. The term "reduction" or "reducing" in connection with tumour size is covered by the term "inhibition" or "inhibit". The reduction of the solid tumour refers to a reduction of the tumour volume. For example, a 10% reduction of a solid tumour means that the volume of the treated tumour has been reduced with 10%.

An important aspect of the present invention lies in the therapeutic application of the solid pharmaceutical formulation of the invention.

Thus, the present invention is also directed to a solid pharmaceutical formulation of the invention for use as a medicament. In particular, the present invention is directed to use of the solid pharmaceutical formulation of the invention for the manufacture of a medicament for treatment of cancer. Alternatively stated, the present invention is directed to a method of treating cancer, said method comprising administering a therapeutically effective amount of the solid pharmaceutical formulation of the invention to a patient in need thereof.

In the present context the term "treatment of a cancer" or "treating a mammal having a cancer" is intended to mean that the solid pharmaceutical formulation described herein is administered in a therapeutically effective amount which is sufficient to i) inhibit growth of the tumour, *ii)* facilitate tumour regression, i.e. reduce the size of the tumour, *iii)* remove the tumour and/or *iv)* inhibit cancer cell metastasis.

The solid pharmaceutical formulation will be administered to patients in a "therapeutically effective" dose, i.e. in a dose that is sufficient to produce the desired effects in relation to the condition for which it is administered. The exact dose will depend on the cancer form to be treated, and will be ascertainable by one skilled in the art using known techniques. A suitable dose of the drug substance, in particular pynasunate, is contemplated to be in the range of about 1-2 g/patient/day, such as 1-1.5 g/patient/day, e.g. 1.3-1.4 g/patient/day, in particular 1.34 g/patient/day.

In a very interesting embodiment of the invention said cancer is a carcinoma, such as a carcinoma selected from the group consisting of malignant melanoma, basal cell carcinoma, ovarian carcinoma, breast carcinoma, non-small cell lung cancer, renal cell carcinoma, bladder carcinoma, recurrent superficial bladder cancer, stomach carcinoma, prostatic carcinoma, pancreatic carcinoma, lung carcinoma, cervical carcinoma, cervical dysplasia, laryngeal papillomatosis, colon carcinoma, colorectal carcinoma and carcinoid tumours, in particular selected from the group consisting of malignant melanoma, non-small cell lung cancer, breast carcinoma, colon carcinoma and renal cell carcinoma.

Thus, in one embodiment of the invention said cancer is malignant melanoma, such as superficial spreading melanoma, nodular melanoma, lentigo maligna melanoma, acral melagnoma, amelanotic melanoma or desmoplastic melanoma. In another embodiment of the invention said cancer is non-small cell lung cancer. In still another embodiment of the invention said cancer is breast carcinoma. In a further embodiment of the invention said cancer is colon carcinoma. In an even further embodiment of the invention said cancer is renal cell carcinoma.

In a further interesting embodiment of the invention said cancer is a sarcoma, such as a sarcoma selected from the group consisting of osteosarcoma, Ewing's sarcoma, chondrosarcoma, malignant fibrous histiocytoma, fibrosarcoma and Kaposi's sarcoma.

In an even further interesting embodiment of the invention said cancer is a glioma.

As will be understood by the skilled person, the above-mentioned cancer types (i.e. carcinomas, sarcomas and gliomas) are characterised by the presence of solid tumours.

In an even further interesting embodiment of the invention said cancer is a lymphoma, such as a lymphoma selected from the group consisting of Hodgkin's disease, non-Hodgkin's disease, B-cell lymphoma, T-cell lymphoma, follicular lymphoma, Burkitt's lymphoma and mycosis fungoides.

In still another interesting embodiment of the invention said cancer is a myeloma, such as multiple myeloma.

One important use in cancer therapy is the reduction and blockade of ascites formation in abdominal tumours of different origin (e.g. uterine endometrium, ovarial cancer, colon cancer) also for mesothelioma.

In addition to its use as anticancer agent the formulation of the invention can be used for other diseases characterised by overshooting angiogenesis, like macular degeneration due to vessel in-growth, corneal transplatation due to lymphatic vessel ingrowth and thereby breakage of the immune privilege. It is helpful in diseases like rheumatoid arthritis with vessels growing ectopically towards the joints. As mentioned above it is effective in asthma. Also diseases with female cycle associated vessel growth are influenced beneficially as is the case for endometriosis.

It will be understood that an even more effective treatment of the various cancer forms may be obtained by combination therapy where the solid pharmaceutical formulation of the invention is combined with a suitable chemotherapeutic agent and/or is combined with radiotherapy and/or is combined with surgery.

Thus, a further aspect the present invention relates to the use of a solid pharmaceutical formulation of the invention for the manufacture of a medicament for treatment of cancer in combination with a chemotherapeutic agent. Analogously, a further aspect of the present invention relates to a method of treating cancer, said method comprising administering a therapeutically effective amount of the solid pharmaceutical formulation of the invention and a chemotherapeutic agent to a patient in need thereof.

Specific examples of suitable chemotherapeutic agents include chemotherapeutic agents selected from the group consisting of adrenocorticosteroids, such as prednisone, dexamethasone or decadron; altretamine (hexalen, hexamethylmelamine (HMM)); amifostine (ethyol); aminoglutethimide (cytadren); amsacrine (M-AMSA); anastrozole (arimidex); androgens, such as testosterone; asparaginase (elspar); bacillus calmette-gurin; bicalutamide (casodex); bleomycin (blenoxane); busulfan (myleran); carboplatin (paraplatin); carmustine (BCNU, BiCNU); chlorambucil (leukeran); chlorodeoxyadenosine (2-CDA, cladribine, leustatin); cisplatin (platinol); cytosine arabinoside (cytarabine); dacarbazine (DTIC); dactinomycin (actinomycin-D, cosmegen); daunorubicin (cerubidine); docetaxel (taxotere); doxorubicin (adriomycin); epirubicin; estramustine (emcyt); estrogens, such as diethylstilbestrol (DES); etopside (VP-16, VePesid, etopophos); fludarabine (fludara); flutamide (eulexin); 5-FUDR (floxuridine); 5-fluorouracil (5-FU); gemcitabine (gemzar); goserelin (zodalex); herceptin (trastuzumab); hydroxyurea (hydrea); idarubicin (idamycin); ifosfamide; IL-2 (proleukin, aldesleukin); interferon alpha (intron A, roferon A); irinotecan (camptosar); leuprolide (lupron); levamisole (ergamisole); lomustine (CCNU); mechlorathamine (mustargen, nitrogen mustard); melphalan (alkeran); mercaptopurine (purinethol, 6-MP); methotrexate (mexate); mitomycin-C (mutamucin); mitoxantrone (novantrone); octreotide (sandostatin); pentostatin (2-deoxycoformycin, nipent); plicamycin (mithramycin, mithracin); prorocarbazine (matulane); streptozocin; tamoxifin (nolvadex); taxol (paclitaxel); teniposide (vumon, VM-26); thiotepa; topotecan (hycamtin); tretinoin (vesanoid, all-trans retinoic acid); vinblastine (valban); vincristine (oncovin) and vinorelbine (navelbine).

Other chemotherapeutic agents, which may be useful for the purposes described herein include the chemotherapeutic agents mentioned in column 12, line 62 to column 13, line 42 of US 6,482,802. For a description of these and other chemotherapeutic agents, see The Merck Index, 12th edition, pp. THER 13-14.

It will be understood that the chemotherapeutic agent is selected under due consideration of the actual cancer form. In a preferred embodiment of the invention the following chemotherapeutic agents are used (together with the solid pharmaceutical formulation described herein) for treatment of the following specific cancer forms: malignant melanoma and cisplatin; malignant melanoma and IL-2; renal cell carcinoma and doxorubicin; renal cell carcinoma and IL-2; breast carcinoma and doxorubicin; breast carcinoma and taxol; colon carcinoma and 5-fluorouracil; colon carcinoma and cisplatin; and non-small cell lung cancer and cisplatin.

The invention is further described in the following examples. The examples should not, in any manner, be understood as limiting the generality of the present specification and claims.

### MATERIALS AND METHODS

### Determination of particle size distribution

Determination of the particle size distribution of pynasunate was performed by laser diffraction using the following equipment and settings:

| | |
|---|---|
| Apparatus: | Sympatec Helos (H0583) |
| Dispersion system: | Aerial dry dispersion using Sympatec Rodos module |
| Focal length: | 500 mm |
| Volume of air stream: | 2 m³/h |
| Prepressure: | 2 bar |
| Dispersion pressure: | 3 bar |
| Lens: | R5 (4.5-875 µm) |
| Optical concentration: | 0.8 - 20% |
| Measurement time: | 2 seconds |
| Optical model: | Fraunhofer under the presumption of spherical particles. |

### Determination of granule coarseness

Determination of the granule coarseness was performed by sieve analysis in accordance with Ph. Eur. Method 2.9.12 using a sieve with a mean mesh width of 250 µm.

### Dissolution method

Determination of pynasunate dissolution from film coated tablets was performed in accordance with the USP 28 Paddle Method using the following test parameters:

| | |
|---|---|
| Apparatus: | USP dissolution apparatus 2, with six covered glass vessels and paddle stirrers |
| Medium: | 0.05 M phosphate buffer (KH₂PO_{4/}HCl), degassed. The pH value was finally adjusted to 3.00±0.05 |
| Filling volume: | 1000 ml |
| Temperature: | 37°C±0.5°C |
| Stirring rate: | 50 rpm±2 rpm |
| Sampling times: | 5, 10, 15, 30, 45 and 60 minutes |
| Detection: | UV-measurement at 316 nm |

### Powder rheometer measurements

For the powder rheometer analysis the following system and parameters were used:

| | |
|---|---|
| Manufacturer: | Freeman Technology |
| System: | FT4 |
| Sample vessel: | 25 ml |
| Blade size: | 23.5 mm |
| Sample preparation: | Conditioning run, dosing of 25 ml sample, weighing of sample |
| Sample run: | Conditioning run and measurement run at 100 mm/s tip speed. 8 times repetition |
| | Conditioning run and measurement run at 70 mm/s tip speed |
| | Conditioning run and measurement run at 40 mm/s tip speed |
| | Conditioning run and measurement run at 10 mm/s tip speed |
| Measurement cycle: | Five sample runs on the same sample |

### EXAMPLES

### Example 1: Drug substance with a small particle size

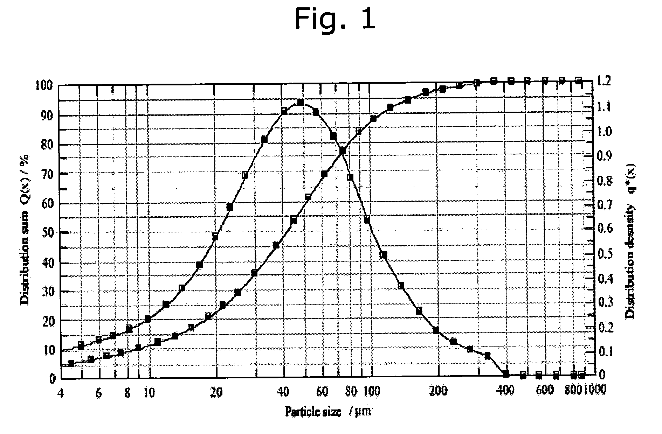

The particle size distribution of a drug batch was analysed as described above. The result of the particle size distribution measurement is given in Figure 1. The following parameters were found: d₅₀ = 42 µm, d₉₀ = 119 µm, d₉₅ = 164 µm and d₉₉ of 282 µm. Film coated tablets were manufactured according to Example 5 below. The film coated tablets showed a rapid dissolution with 93% of active drug substance being dissolved after 30 minutes using the USP 28 Paddle Method.

### Example 2: Drug substance with a medium particle size

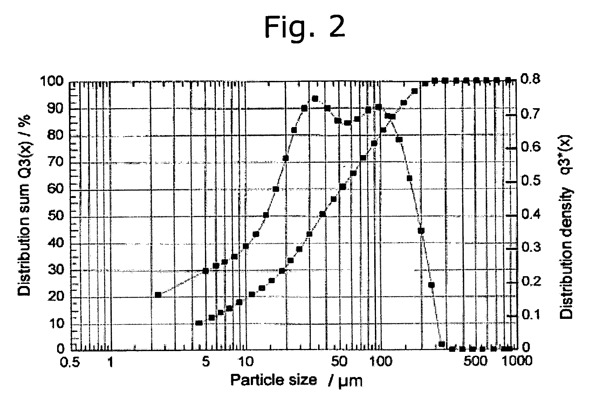

The particle size distribution of a drug batch was analysed as described above. The result of the particle size distribution measurement is given in Figure 2. The following parameters were found: d₅₀ = 37 µm, d₉₀ = 141 µm, d₉₅ = 174 µm and d₉₉ of 229 µm. Film coated tablets were manufactured according to Example 5 below. The film coated tablets showed a rapid dissolution with 87% of active drug substance being dissolved after 30 minutes using the USP 28 Paddle Method.

### Example 3: Drug substance with a large particle size

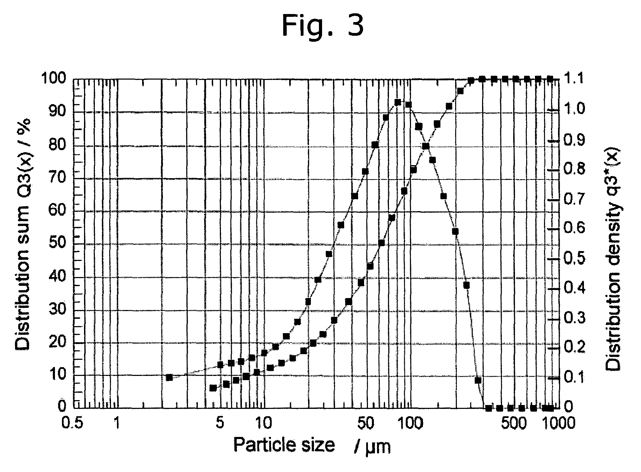

The particle size distribution of a drug batch was analysed as described above. The result of the particle size distribution measurement is given in Figure 3. The following parameters were found: d₅₀ = 63 µm, d₉₀ = 169 µm, d₉₅ = 202 µm and d₉₉ of 247 µm. Film coated tablets were manufactured according to Example 5 below. The film coated tablets showed a rapid dissolution with 78% of active drug substance being dissolved after 30 minutes using the USP 28 Paddle Method.

### Example 4: Drug substance with a very large particle size

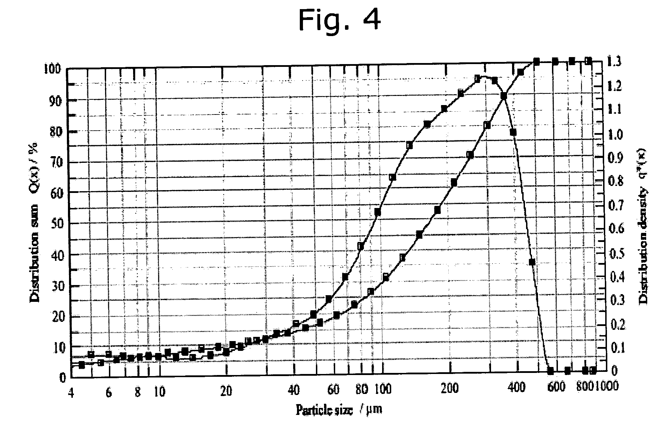

The particle size distribution of a drug batch was analysed as described above. The result of the particle size distribution measurement is given in Figure 4. The following parameters were found: d₅₀ = 170 µm, d₉₀ = 374 µm, d₉₅ = 419 µm and d₉₉ of 491 µm. Film coated tablets were manufactured according to Example 5 below. The film coated tablets showed a slow dissolution with only 58% of active drug substance being dissolved after 30 minutes using the USP 28 Paddle Method.

### Example 5: Manufacturing of high-load pynasunate tablets

Tablets were prepared from the following ingredients:

| Ingredient | Amount (kg) |
|---|---|
| Homogenised pynasunate | 26.800 |
| Lactose monohydrate | 14.080 |
| Hydroxypropylmethylcellulose (HPMC) | 1.120 |
| Water | 12.380 |
| Croscarmellose sodium | 1.200 |
| Magnesium stearate | 0.800 |

44 kg of granules were obtained by the following process: Lactose and pynasunate were charged into a fluid bed granulator. The granulation process was started and the binder solution, prepared by dissolving HPMC in water, was spray on the fluidised bed. At the end of the granulation process croscarmellose sodium and magnesium stearate were added to the fluid bed granulator and blended with the granules.

132 kg of tablets were obtained by the following process: The granulation process was performed three times after which the granules were collected in an appropriate hopper which was then applied to a rotary press.

The granules were compressed to oblong tablets (17.6 x 7 curvature 4 mm) of 550 mg containing the following ingredients:

| Ingredient | Amount (mg) |
|---|---|
| Pynasunate | 335 (60.9%) |
| Lactose monohydrate | 176 |
| Hydroxypropylmethylcellulose (HPMC)* | 14 |
| Croscarmellose sodium | 15 |
| Magnesium stearate | 10 |
| Total | 550 |

The tablets were film-coated with a dispersion containing the following ingredients:

| Ingredient | Amount (kg) |
|---|---|
| Hydroxypropylmethylcellulose (HPMC) | 1.820 |
| Talc | 0.364 |
| Iron oxide yellow pigment | 0.425 |
| Titanium dioxide | 0.991 |
| Water I | 12.237 |
| Water II | 6.117 |

Iron oxide yellow pigment, talc and titanium dioxide were suspended in water II and added to a solution of HPMC in water I. Additional water was added to a final weight of 23.994 kg. The dispersion was homogenised in a colloidal mill and subsequently sprayed onto the tablets. Coating was performed until the film-coated tablets had reached a weight of 562 mg each.

### Example 6: Manufacturing of very high-load pynasunate tablets

Tablets were prepared from the following ingredients:

| Ingredient | Amount (kg) |
|---|---|
| Homogenised pynasunate | 39.179 |
| Lactose monohydrate | 1.263 |
| Hydroxypropylmethylcellulose (HPMC) | 1.123 |
| Water | 12.380 |
| Croscarmellose sodium | 1.628 |
| Magnesium stearate | 0.807 |

44 kg of granules were obtained by the following process: Lactose and pynasunate were charged into a fluid bed granulator. The granulation process was started and the binder solution, prepared by dissolving HPMC in water, was spray on the fluidised bed. At the end of the granulation process croscarmellose sodium and magnesium stearate were added to the fluid bed granulator and blended with the granules.

132 kg of tablets were obtained by the following process: The granulation process was performed three times after which the granules were collected in an appropriate hopper which was then applied to a rotary press.

The granules were compressed to droplet-shaped tablets (18 x 6 curvature 12 mm) of 627 mg containing the following ingredients:

| Ingredient | Amount (mg) |
|---|---|
| Pynasunate | 558.3 (89.0%) |
| Lactose monohydrate | 18.0 |
| Hydroxypropylmethylcellulose (HPMC) | 16.0 |
| Croscarmellose sodium | 23.2 |
| Magnesium stearate | 11.5 |
| Total | 627.0 |

The tablets from were film-coated with a dispersion containing the following ingredients:

| Ingredient | Amount (kg) |
|---|---|
| Hydroxypropylmethylcellulose (HPMC) | 1.620 |
| Talc | 0.324 |
| Iron oxide yellow pigment | 0.378 |
| Titanium dioxide | 0.882 |
| Water I | 10.891 |
| Water II | 5.444 |

Iron oxide yellow pigment, talc and titanium dioxide were suspended in water II and added to a solution of HPMC in water I. Additional water was added to a final weight of 23.994 kg. The dispersion was homogenised in a colloidal mill and subsequently sprayed onto the tablets. Coating was performed until the film-coated tablets had reached a weight of 640 mg each.

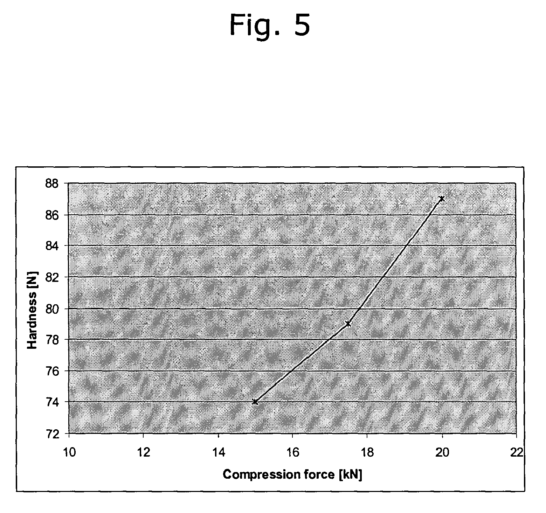

The very high-load film-coated pynasunate tablets exhibited excellent physical stability, cf. the compression force vs. hardness data shown in Fig. 5.

### Example 7: Optimised high-load film-coated pynasunate tablet

Pynasunate particles having a d₅₀ of 64 µm and a d₉₀ of 173 µm were processed to granules as described in Example 5. 47% of the granules were coarser than 250 µm. The compression process showed no sticking of the granules to the punches and the resulting tablets showed excellent physical stability and no capping. The film-coated tablets released 85.8% of the pynasunate after 30 minutes using the USP 28 Paddle Method described above.

### Example 8: Compression of pure pynasunate

54 g of homogenised, non-micronized, pynasunate was mixed with 1 g of magnesium stearate. The blend was compressed to tablets of 550 mg weight each using a single punch compressing machine. The compression process was not successful. The material sticked to the punches at all available compression forces and speeds. No tablets were obtained.

### Example 9: Manufacturing of very high loaded pynasunate pellets

1675g of of homogenised, non-micronized, pynasunate was mixed with 52g of mannitol and 96g of microcrystalline cellulose. To this blend 1130ml of water was added. The mixture was given into an extruder and extruded through an 1000 µm round shaped grid. The extrudates were shaped to round pellets on a spheronizer and then dried to equilibrium in an fluid bed drier at an inlet air temperature of 60°C. After that the pellets were conditioned to ambient conditions by open storage for 12 hours. The pellets released 89.9% of the pynasunate after 30 minutes using the USP 28 Paddle Method described above.

## Claims

1. A solid pharmaceutical formulation comprising at least 50% by weight of the total formulation of non-micronized (4-chlorophenyl)[4-(4-pyridylmethyl)-phthalazin-1-yl], or a pharmaceutically acceptable salt thereof, wherein at least 70% of said (4-chlorophenyl)[4-(4-pyridylmethyl)-phthalazin-1-yl], or a pharmaceutical acceptable salt thereof, is dissolved from said solid pharmaceutical formulation within 30 minutes as determined by the USP 28 Paddle Method using 0.05 M KH₂PO₄/HCl buffer adjusted to pH 3.0 at 37°C as the dissolution media and 50 rpm as the stirring rate.

2. The formulation according to claim 1, wherein at least 75%, preferably at least 80%, of said non-micronized (4-chlorophenyl)[4-(4-pyridylmethyl)-phthalazin-1-yl], or a pharmaceutically acceptable salt thereof, is dissolved from said solid pharmaceutical formulation within 30 minutes.

3. The formulation according to claim 1 or 2, wherein said non-micronized (4-chlorophenyl)[4-(4-pyridylmethyl)-phthalazin-1-yl], or a pharmaceutically acceptable salt thereof, has a d₉₀ value of at least 60 µm when determined as described herein.

4. The formulation according to claim 3, wherein said d₉₀ value is at least 70 µm, such as at least 80 µm, e.g. at least 90 µm, preferably at least 100 µm, such as at least 110 µm, e.g. at least 120 µm, more preferably at least 130 µm, such as at least 140 µm.

5. The formulation according to any of the preceding claims, wherein said non-micronized (4-chlorophenyl)[4-(4-pyridylmethyl)-phthalazin-1-yl], or a pharmaceutically acceptable salt thereof, has a d₉₀ value in the range of 50-300 µm when determined as described herein.

6. The formulation according to claim 5, wherein said d₉₀ value is in the range of 60-250 µm, such as in the range of 70-200 µm, e.g. in the range of 80-200 µm, preferably in the range of 90-200 µm, such as in the range of 100-200 µm, e.g. in the range of 110-190 µm, more preferably in the range of 120-180 µm, such as in the range of 130-170 µm, e.g. in the range of 140-160 µm.

7. The formulation according to any of the preceding claims, wherein said non-micronized (4-chlorophenyl)[4-(4-pyridylmethyl)-phthalazin-1-yl], or a pharmaceutically acceptable salt thereof, has a d₅₀ value of at least 15 µm when determined as described herein.

8. The formulation according to claim 7, wherein said d₅₀ value is at least 20 µm, such as at least 25 µm, e.g. at least 30 µm, preferably at least 35 µm.

9. The formulation according to any of the preceding claims, wherein said non-micronized (4-chlorophenyl)[4-(4-pyridylmethyl)-phthalazin-1-yl], or a pharmaceutically acceptable salt thereof, has a d₅₀ value in the range of 15-100 µm when determined as described herein.

10. The formulation according to claim 9, wherein said d₅₀ value is in the range of 20-90 µm, such as in the range of 25-80 µm, e.g. in the range of 30-70 µm.

11. The formulation according to any of the preceding claims, comprising at least 55% by weight of the total formulation of non-micronized (4-chlorophenyl)[4-(4-pyridylmethyl)-phthalazin-1-yl], or a pharmaceutically acceptable salt thereof.

12. The formulation according to claim 11, comprising at least 60% by weight, such as at least 65% by weight, e.g. at least 70% by weight, preferably at least 75% by weight, such as at least 80% by weight, e.g. at least 85% by weight, at least 90% by weight or at least 95% by weight of the total formulation of non-micronized (4-chlorophenyl)[4-(4-pyridylmethyl)-phthalazin-1-yl], or a pharmaceutically acceptable salt thereof.

13. The formulation according to any of the preceding claims further comprising at least one pharmaceutically acceptable excipient.

14. The formulation according to claim 13, wherein said at least one pharmaceutically acceptable excipient is selected from the group consisting of fillers, binders, surfactants, disintegrants, glidants and lubricants.

15. The formulation according to any of the preceding claims, wherein said (4-chlorophenyl)[4-(4-pyridylmethyl)-phthalazin-1-yl] is in the form of a pharmaceutically acceptable salt thereof.

16. The formulation according to claim 15, wherein said pharmaceutically acceptable salt thereof is an acid addition salt.

17. The formulation according to claim 16, wherein said acid addition salt is formed from an inorganic acid or an organic acid.

18. The formulation according to claim 17, wherein said pharmaceutically acceptable salt of (4-chlorophenyl)[4-(4-pyridylmethyl)-phthalazin-1-yl] is (4-chlorphenyl)[4-(4-pyridylmethyl)-phtalazin-1-yl]ammonium hydrogen succinate.

19. The formulation according to any of the preceding claims, wherein said formulation is a granule.

20. The formulation according to any of the preceding claims, wherein said formulation is a tablet.

21. A solid dosage form comprising a solid pharmaceutical formulation according to any of the preceding claims.

22. A solid dosage form according to claim 21, which is in a unit dosage form.

23. A solid dosage form according claim 21, which is in a multiple unit dosage form.

24. A solid dosage form according claim 23, wherein the dosage form are pellets.

25. The solid unit dosage form according to claim 21, wherein said solid unit dosage form is adapted for oral administration.

26. The solid unit dosage form according to claim 22, wherein said solid unit dosage form is in the form of a tablet, a capsule or sachet.

27. The tablet according to claim 26, wherein said solid unit dosage form is in the form of a tablet

28. The tablet according to claim 27, wherein said tablet is coated.

29. The tablet according to claim 28, wherein said tablet is film-coated.

30. The tablet according to any of claims 26-29, wherein said tablet has a weight in the range of 500-700 mg.

31. The tablet according to claim 30, wherein said tablet has a weight in the range of 525-675 mg, such as in the range of 525-650 mg, e.g. in the range of 525-575 mg or in the range of from 610-650 mg.

32. The tablet according to any of claims 26-31, wherein said tablet comprises 300-600 mg (4-chlorphenyl)[4-(4-pyridylmethyl)-phtalazin-1-yl]ammonium hydrogen succinate.

33. The tablet according to claim 32, wherein said tablet comprises 300-350 mg (4-chlorphenyl)[4-(4-pyridylmethyl)-phtalazin-1-yl]ammonium hydrogen succinate.

34. The tablet according to claim 33, wherein said tablet comprises 310-350 mg, such as 320-350 mg, e.g. 330-340 mg, preferably about 335 mg (4-chlorphenyl)[4-(4-pyridylmethyl)-phtalazin-1-yl]ammonium hydrogen succinate.

35. The tablet according to claim 34, wherein said tablet comprises 335 mg (4-chlorphenyl)[4-(4-pyridylmethyl)-phtalazin-1-yl]ammonium hydrogen succinate, 176 mg lactose monohydrate, 14 mg hydroxypropylmethylcellulose, 15 mg croscarmellose sodium and 10 mg magnesium stearate.

36. The tablet according to claim 32, wherein said tablet comprises 500-600 mg, such as 510-600 mg, e.g. 520-600 mg, preferably 530-590 mg, such as 540-580 mg, e.g. 550-570, more preferably 555-565 mg, such as about 558.3 mg (4-chlorphenyl)[4-(4-pyridylmethyl)-phtalazin-1-yl]ammonium hydrogen succinate.

37. The tablet according to claim 36, wherein said tablet comprises 558.3 mg (4-chlorphenyl)[4-(4-pyridylmethyl)-phtalazin-1-yl]ammonium hydrogen succinate, 18 mg lactose monohydrate, 16 mg hydroxypropylmethylcellulose, 23.2 mg croscarmellose sodium and 11.5 mg magnesium stearate.

38. A process for the preparation of granules comprising at least 50% by weight of the total granule of non-micronized (4-chlorophenyl)[4-(4-pyridylmethyl)-phthalazin-1-yl], or a pharmaceutically acceptable salt thereof, said process comprising the steps of
i) preparing a liquid medium comprising the binder
ii) subjecting a powder mixture of excipients such as filler and diluents and at least 50% by weight of the components of non-micronized (4-chlorophenyl)[4-(4-pyridylmethyl)-phthalazin-1-yl], or a pharmaceutically acceptable salt thereof to a granulator
iii) subjecting said liquid medium to a granulation process; and
iv) optionally extruding and shaping the granules
v) optionally drying the granules
vi) optionally adding further excipients such as disintegrants and lubricants to the granules
vii) optionally collecting the granules.

39. The process according to claim 38, wherein said powder blend comprises at least 55% by weight of the components of the medium, excluding liquid, of non-micronized (4-chlorophenyl)[4-(4-pyridylmethyl)-phthalazin-1-yl], or a pharmaceutically acceptable salt thereof.

40. The process according to claim 39, wherein said powder blend comprises at least 60% by weight, such as at least 65% by weight, e.g. at least 70% by weight, preferably at least 75% by weight, such as at least 80% by weight, e.g. at least 85% by weight, at least 90% by weight or at least 95% by weight of
non-micronized (4-chlorophenyl)[4-(4-pyridylmethyl)-phthalazin-1-yl], or a pharmaceutically acceptable salt thereof.

41. The process according to any of claims 38-40, wherein said non-micronized (4-chlorophenyl)[4-(4-pyridylmethyl)-phthalazin-1-yl], or a pharmaceutically acceptable salt thereof, is as defined in any of claims 3-10.

42. The process according to any of claims 38-41, wherein said liquid medium comprises a binder.

43. The process according to any of claims 38-42, wherein said liquid medium does not contain a disintegrant.

44. The process according to any of claims 38-43, wherein said liquid medium does not contain a lubricant.

45. The process according to any of claims 38-44, wherein a disintegrant is added during, preferably at the end of, the granulation process.

46. The process according to any of claims 38-45, wherein a lubricant is added during, preferably at the end of, the granulation process.

47. The process according to any of claims 38-46, wherein said liquid is water.

48. The process according to any of claims 38-47, wherein said granulation process is performed by means of high shear granulation, fluid bed drying, fluid bed granulation, roller compaction or extrusion.

49. The process according to claim 48, wherein said granulation process is performed by fluid bed granulation.

50. The process according to any of claims 38-49, wherein at least 25% of the granules are coarser than 250 µm as determined by sieve analysis in accordance with Ph. Eur. Method 2.9.12 using a sieve with a mean mesh width of 250 µm.

51. The process according to claim 50, wherein at least 30% of the granules, such as at least 35% of the granules, e.g. at least 40% of the granules are coarser than 250 µm.

52. The process according to any of claims 38-51, wherein 25-70% of the granules are coarser than 250 µm as determined by sieve analysis in accordance with Ph. Eur. Method 2.9.12 using a sieve with a mean mesh width of 250 µm.

53. The process according to claim 52, wherein 30-70% of the granules, such as 35-70% of the granules, e.g. 40-70% of the granules, preferably 40-65% of the granules, such as 40-60% of the granules are coarser than 250 µm.

54. Granules obtainable by the process according to any of claims 38-53.

55. A process for the preparation of a solid unit dosage form according to claim 22, said process comprising the steps of
i) preparing granules comprising at least 50% by weight of the total granule of non-micronized (4-chlorophenyl)[4-(4-pyridylmethyl)-phthalazin-1-yl], or a pharmaceutically acceptable salt thereof, according to any of claims 38-53;
ii) formulating said granules into solid unit dosage forms.

56. The process according to claim 55, wherein said solid unit dosage form is as defined in any of claims 25-37.

57. A solid unit dosage form obtainable by the process according to claim 55 or 56.

58. A composition of non-micronized (4-chlorphenyl)[4-(4-pyridylmethyl)-phtalazin-1-yl]ammonium hydrogen succinate particles having a d₉₀ value in the range of 50-300 µm and a d₅₀ value in the range of 15-100 µm, when determined as described herein.

59. The composition according to claim 58, wherein the d₉₀ value is in the range of 100-200 µm and the d₅₀ value is in the range of 30-70 µm, when determined as described herein.

60. The composition according to claim 59, wherein d₅₀ value, the d₉₀ value, the d₉₅ value and the d₉₉ value are as defined in Example 1, Example 2 or Example 3 herein.

61. A formulation according to any of claims 1-20, a dosage form according to any of claims 21-37 or 57, or a composition according to any of claims 58-60, for use as a medicament.

62. Use of a formulation according to any of claims 1-20, use of a dosage form according to any of claims 21-37 or 57, or use of a composition according to any of claims 58-60 for the manufacture of a medicament for the treatment of cancer.

63. A method of treating cancer, said method comprising administering a therapeutically effective amount of the formulation according to any of claims 1-20, a dosage form according to any of claims 21-37 or 57, or a composition according to any of claims 58-60, to a patient in need thereof.
